# EUROPEAN PATENT SPECIFICATION

(11) **EP 4 108 095 B1**
(45) Date of publication and mention of the grant of the patent: **04.03.2026**
(21) Application number: 21382550.8
(22) Date of filing: 22.06.2021
(51) Int. Cl.: A23K 10/00, A61K 9/16, A61K 9/50, A61K 31/197, A61P 25/00, A61P 25/22

(54) **MICROCAPSULES CONTAINING GAMMA-AMINOBUTYRIC ACID**
GAMMA-AMINOBUTTERSÄURE-HALTIGE MIKROKAPSELN
MICROCAPSULES CONTENANT UN ACIDE GAMMA-AMINOBUTYRIQUE

(43) Date of publication of application: 28.12.2022
(73) Proprietor: Fundación Tecnalia Research & Innovation, 20009 San Sebastian (ES)
(72) Inventor: CHÁVARRI HUEDA, María Blanca, 01510 MIÑANO (ES); MARAÑÓN GARCÍA, Izaskun, 01510 MIÑANO (ES); DIEZ GUTIÉRREZ, Lucía Camino, 01510 MIÑANO (ES)
(74) Representative: ZBM Patents - Zea, Barlocci & Markvardsen

(56) References cited:
- EP-A1- 3 205 216
- CN-A- 102 125 168
- JP-A- 2017 073 991
- PANDEY POOJA ET AL: "Co-microencapsulation of [gamma]-aminobutyric acid (GABA) and probiotic bacteria in thermostable and biocompatible exopolysaccharides matrix", LWT- FOOD SCIENCE AND TECHNOLOGY, ACADEMIC PRESS, UNITED KINGDOM, vol. 136, 29 September 2020 (2020-09-29), XP086388154, ISSN: 0023-6438, [retrieved on 20200929], DOI: 10.1016/J.LWT.2020.110293

## Description

The present invention relates to the field of biologically-active compounds microencapsulation. In particular, the invention relates to microcapsules comprising gamma-aminobutyric acid and one or more probiotics that are able to reach the intestine in an intact form.

### BACKGROUND ART

GABA, gamma-aminobutyric acid, is an amino acid with a non-proteinic structure mainly produced by plants, animals and microorganisms This amino acid performs different functions depending on the producer organism. For instance, GABA is a well-known inhibitory neurotransmitter effective in the central nervous system (CNS) of animals, however, in plants and microorganisms, it is synthesized as a protective mechanism against stressful situations.

The biotechnological field currently presents an emerging interest in GABA such as its importance in the synthesis of nylon 4 which is considered a potential biodegradable polymer. Moreover, bioremediation of acid mine drainage is another important area involved in GABA production. As the biosynthetic pathway of this molecule is considered as an essential mechanism to protect against low pH stress, it is a promising substitute to the addition of chemicals for the neutralization of the acidic environment.

Nevertheless, pharmaceutic and food industries are a landmark in the biotechnological cluster due to the intensive research developing food supplements or fermented foods rich in GABA, respectively. This kind of products takes in advance the huge amount of beneficial health effects of this amino acid such as gut modulation, neurostimulation or cardioprotection. In the beginning, chemical synthesis was used to cover the demand of GABA, but the use of microorganisms to produce this compound replaced the chemical process due to the high yield, low price and lower environmental pollution of the biosynthetic process.

GABA is an important postbiotic (i.e. molecule produced by different bacteria as a result of their metabolism) considered as an inhibitory neurotransmitter that has gained interest over the years due to its essential role in the nervous system. The inhibitory effect of GABA is performed by binding to the GABAergic receptor system composed of three specific receptors: GABA_{A}, GABA_{B}, and GABA_{C}. Through these receptors, GABA can modulate moods like relaxation, sleep disorders or temporal and spatial memory. More beneficial effects have been demonstrated in epilepsy, depression, diabetes, asthmatic disorders or cancer. Moreover, several researches have proved the importance of GABA in the development of some neural diseases such as schizophrenia, Alzheimer's disease, Parkinson's disease or Huntington's disease, as detailed below.

In the same way than probiotics and prebiotics, GABA and other postbiotic compounds are frequently added to food or feed products to provide what is known as functional foods or feeds. Functional food or feed is a natural or processed food or feed that contains known biologically-active components which when in defined quantitative and qualitative amounts provides a clinically proven and documented health benefit. A common difficulty associated with the incorporation of certain substances in food or feed products is the loss of their activity, due decomposition and/or destruction. These problems usually emerge during the food manufacturing process, during the product shelf life and/or during the passage of these components through the digestive tract of the consumer (host). Most of functional substances are sensitive to most types of conventional food processing techniques, such as high temperatures, milling, mixing, baking or extrusion. Even during the shelf life of food product, it is difficult to assure the stability or activity of these components since they could interact with other food components, such as metal chelators, surfactants, oxidants, etc. Maintenance of the active form of GABA and other functional substances through the industrial process, storage and passage through the gastro-intestinal tract is essential in order to achieve their beneficial healthy effects in the host. Thus, these substances (i.e. GABA) must reach the gut in sufficient amount and unaltered to be absorbed and to exert their effect.

Microencapsulation is well-known as a suitable method to protect some labile substances, and/or to control their release in the desired intestine portion. With this technology a micro-environment is created, were encapsulated substances are protected from non-favourable external conditions, such as oxidative processes, but also water, heat, or light are not convenient for the compound stability.

Microcapsules containing GABA are disclosed for example in the Chinese patent CN102125168 (HANGZHOU KANGDEQUAN FEED CO LTD), where a method for preparing rumen bypass gamma-aminobutyric acid in form of microcapsules is disclosed. The gamma-aminobutyric acid is microencapsulated and coated with starch and microcellulose, so that it will not be fermented or degraded when passing through the rumen of ruminants and will reach intact the intestinal tract. Effective release increases its effective absorption and utilization in the intestinal tract, and fully exert its physiological functions. The microcapsules are said to contain (calculated by weight percentage) starch 5-20%, hydroxypropyl methylcellulose 5-10%, gamma-aminobutyric acid powder 50-80%, glyceryl stearate 5-15% and resin 2# 3-5%.

The Japanese patent application JP2017073991 ((AOMORI PREFECTURAL IND TECH RES CENTER CALORIA JAPAN CO LTD) also discloses microcapsules with GABA. They are edible microcapsules containing, a core substance mainly composed of gamma-aminobutyric acid, and a coating including a membrane substance, in particular protein. The membrane substance is coating the core with multiple layers. In some particular examples, the membrane material comprises an inner layer comprising zein and an outer layer comprising albumin covering the inner layer. The microcapsules are proposed for preventing the leakage of GABA and for suppressing morphological changes even if it is added to a food with high salt content.

Finally, microcapsules containing GABA and a strain of *Lactobacillus plantarum* embedded in a mixture of exopolysaccharides containing inulin, dextran and maltodextrin are disclosed in the document of Pandey, P. and Mishra, H.N. (2020), Co-microencapsulation of γ-aminobutyric acid (GABA) and probiotic bacteria in thermostable and biocompatible exopolysaccharide matrix. LWT-Food Science and Technology 136 (2021) 110293. GABA and *Lactobacillus plantarum* encapsulation efficiencies of 84.22% and 99.21%, respectively, were obtained with a shell composition obtained by D-optimal mixture design with 0.4%, 4.6% and 8.4% of inulin, dextran and maltodextrin.

The microcapsules, obtained by spray drying, had a spherical morphology with particle size in the range of 10-35 µm. The microcapsules were highly stable and resistant to high temperatures during the food processing. No significant differences in the viability of bacteria and GABA retention was found after 120 days. Authors propose the microcapsules to offer GABA, probiotics and prebiotics in a single multifunctional platform for the delivery through various food matrices. These microcapsules avoid water absorption in wet environments, and they are thought to release the contents once resuspended in a liquid, as usually is done with nutraceutical supplements or functional food due to the presence of maltodextrin.

Alginic acid and calcium alginate is one of the encapsulating materials certified for food applications as "generally recognized as safe" (GRAS) materials. It is a highly insoluble material, but substances like GABA that are soluble in water diffuse through the alginic polymeric net and are not efficiently retained when alginic-based microcapsules are employed. Thus, although alginate microcapsules are used for the prolonged release of substances that have to achieve the gut, microencapsulation efficiencies of soluble substances are low.

This effect of low efficiency of microencapsulation of GABA in alginate microcapsules is disclosed in more detailed in the document of Polkovnikova, Y. and Korionova, K. (2019) Microcapsules made of sodium alginate for the prolonged release of phenibut, J Res Pharm; vol. no. 23(6), pp.:1040-1047. Polkovnikova, Y. and Korionova, K. disclose microcapsules of the GABA derivate known as phenibut (γ-amino-β-phenylbutyric acid) embedded in alginate and prepared by mixing phenibut with a solution of sodium alginate and extrusion through a sprayer into 0.2M solution of calcium chloride. The authors indicate that the efficiency of microencapsulation was affected by the concentration of the film-forming material (i.e., alginate) and by the ratio of nucleus/polymer. A microencapsulation efficiency of 28.1% was attained with a concentration of sodium alginate solution of 2% and a ratio of nucleus/polymer of 1:1. If the concentration of sodium alginate solution was more than 2% a considerable decrease of efficiency was noticeable as for the ratio of nucleus/polymer 1:1 or 0.5:1.

Multilayer microcapsules containing alginate-based layers are also disclosed in European patent EP3205216 (FUNDACIÓN TECNALIA RESEARCH & INNOVATION). These microcapsules allow the simultaneous encapsulation of incompatible probiotics and/or prebiotics. Encapsulated prebiotics in microcapsules amount from 20 to 40% (e.g., in Example 2, the microcapsule type B with a 38% w/w of fructooligosaccharide in an inner layer of alginate; and the microcapsule type C with a 20% of quercetin, a non-water soluble substance, in an inner layer of alginate).

In view of all the above, there is still a need of microcapsules able to encapsulate high amounts of GABA by a high yield encapsulation process, that can be used in food processing for the preparation of functional foods, and to assure its stability until the microcapsule reach the intestine were GABA is to be absorbed.

### SUMMARY OF THE INVENTION

The inventors have developed novel microcapsules and a method for their preparation which overcome the shortcomings mentioned above.

A first aspect of the invention thus refers to a microcapsule comprising: gamma-aminobutyric acid (GABA) embedded in a hydrogel forming polymer selected from alginate polymer, or a mixture of alginate and pectin, wherein the alginate has such a molecular weight that gives a viscosity that is from 100 mPa.s to lower than 400 mPa.s when measured at 25 °C in a dynamic viscosimeter and in a water solution at a concentration from 10 to 20 g/L of the said alginate polymer.

As will be depicted in the Examples below, these microcapsules allowed an encapsulation efficiency from 60 % to 100 % measured as the ratio between the encapsulated GABA and the initally dissolved and further extruded (see below) GABA.

This encapsulation efficiency is higher than 90% when the microcapsule of the first aspect is further coated with a second or subsequent layers.

Thus, a second aspect of the invention is a multilayer microcapsule, comprising:
(a) a core comprising the microcapsule as defined in the first aspect; and
(b) a coating layer surrounding the core, and comprising a hydrogel forming polymer.

Thus, inventors surprisingly realised that with a hydrogel forming polymer which molecular weight is that giving a viscosity from 100 mPa.s to lower than 400 mPa.s, when measured at 25 °C in a dynamic viscosimeter and in a water solution at a concentration from 10 to 20 g/L, high encapsulation efficiencies of GABA could be achieved. These microcapsules unexpectedly retained high amounts of GABA, which was released in the gut (intestine), as will be illustrated in the examples. This way, the compound is provided in the desired zone of the digestive apparatus.

Not trivial is the obtaining of the microcapsules containing all the features and advantages as defined above, since as indicated microcapsules of the state of the art with alginate and/or pectin do not allow high encapsulation efficiencies of GABA. As a result of extensive research, the inventors have developed a method to prepare the microcapsules of the invention.

The simpler microcapsules of the first aspect are obtained with a particular new process of preparation. Thus, a third aspect of the invention is a process for the preparation of a microcapsule as defined in the first aspect, or in any embodiment of the same, comprising:
(i) preparing a solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3 of GABA:hydrogel forming polymer, wherein when said hydrogel forming polymer is alginate it has a molecular weight that gives a viscosity from 100 mPa.s to lower than 400 mPa.s, when measured at 25 °C in a dynamic viscosimeter and in a water solution at a concentration from 10 to 20 g/L of the said alginate polymer;
(ii) extruding the solution of step (i) through a nozzle with a section (i.e., diameter) from 50 to 500 µm while applying a frequency of vibration to the nozzles from 100 Hz to 1000 Hz to obtain droplets; and
(iii) harvesting the droplets into a divalent ion solution for hardening; and
(iv) optionally drying the microcapsules obtained in step (iii).

Also, the multilayer microcapsules of the second aspect are obtained with a particular new process of preparation. Thus, in a fourth aspect the invention encompasses a process for the preparation of a multilayer microcapsule comprising:
(i) preparing a core (a) solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3 of GABA:hydrogel forming polymer, wherein when said hydrogel forming polymer is alginate it has such a molecular weight that gives a viscosity from 100 mPa.s to lower than 400 mPa.s, when measured at 25 °C in a dynamic viscosimeter and in a water solution at a concentration from 10 to 20 g/L of the said alginate;
(ii) preparing a coating layer (b) solution comprising a hydrogel forming polymer;
(iii) extruding the core solution of step (i) and the coating layer solution of step (iii) through concentric nozzles, each with a section (i.e., diameter) from 50 to 500 µm and having different section or diameters defining an inner nozzle and an external nozzle, while applying a frequency of vibration to the nozzles from 100 Hz to 1000 Hz to obtain multilayer droplets, in such a way that the core solution flows through the inner nozzle and the coating layer solution flows through the external nozzle;
(iv) harvesting the multilayer droplets into a divalent ion solution for hardening; and
(v) optionally drying the microcapsules obtained in step (iv).

The microcapsule of the first aspect may also be defined by its preparation process of preparation. Thus, it is also part of the present invention a microcapsule comprising gamma-aminobutyric acid (GABA) embedded in a hydrogel forming polymer selected from alginate polymer, or a mixture of alginate and pectin, wherein the alginate has such a molecular weight that gives a viscosity from 100 mPa.s to lower than 400 mPa.s, when measured at 25 °C in a dynamic viscosimeter and in a water solution at a concentration from 10 to 20 g/L, said microcapsule obtainable by a process comprising:
i. preparing a solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1.0 to 1:3.0 of GABA:hydrogel forming polymer, wherein when said hydrogel forming polymer is alginate and it has a molecular weight that is one that gives a viscosity from 100 mPa.s to lower than 400 mPa.s, when measured at 25 °C in a dynamic viscosimeter and in a water solution at a concentration from 10 to 20 g/L;
ii. extruding the solution of step (i) through a nozzle with a section (i.e., diameter) from 50 to 500 µm while applying a frequency vibration to the nozzles from 100 Hz to 1000 Hz to obtain droplets; and
iii. harvesting the droplets into a divalent ion solution for hardening; and
iv. optionally drying the microcapsules obtained in step (iii).

In the same way, the multilayer microcapsules of the second aspect may be defined by its process of preparation. Thus, it is also part of the present invention a multilayer microcapsule comprising:
(a) a core comprising the microcapsule as defined in the first aspect; and
(b) a coating layer surrounding the core, and comprising a hydrogel forming polymer, the multilayer microcapsule being obtainable a process comprising:
   (i) preparing a core solution (a) comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1.0 to 1:3.0 of GABA:hydrogel forming polymer, wherein when said hydrogel forming polymer is alginate it has a molecular weight that is one that gives a viscosity from 100 mPa.s to lower than 400 mPa.s, when measured at 25 °C in a dynamic viscosimeter and in a water solution at a concentration from 10 to 20 g/L;
   (ii) preparing a coating layer (b) solution comprising a hydrogel forming polymer;
   (iii) extruding the core solution and the coating layer solution of steps (i) and (ii) through concentric nozzles, each with a section from 50 to 500 µm and having different section or diameters defining an inner nozzle and an external nozzle, while applying a frequency of vibration to the nozzles from 100 Hz to 1000 Hz to obtain multilayer droplets, in such a way that the core solution flows through the inner nozzle and the coating layer solution flows through the external nozzle; and
   (iv) harvesting the multilayer droplets into a divalent ion solution for hardening;
   (v) optionally drying the microcapsules obtained in step (iv).

The microcapsule as defined in any of the first and second aspects can be used for the manufacturing of a food or feed product, a food or feed ingredient, a nutraceutical, a pharmaceutical or a cosmetic composition.

Another additional aspect of the invention is a food or feed product, a food or feed ingredient, a nutraceutical, a pharmaceutical or a cosmetic composition comprising microcapsules as defined in any one of the first and second aspects.

The microcapsules of the invention provide several advantages. These microcapsules surprisingly allow the encapsulation of high amounts of GABA, and they also have an optimal resistance profile, including an ideal combination of processing, storage, and enteric resistance.

As previously indicated, microcapsules with alginate containing layers have until now not been effective for the encapsulation of in-water soluble substances, as GABA is. This is mainly because during microencapsulation GABA tends to rest in, or to diffuse to the water phase of the mixture. Unexpectedly, inventors achieved with this polymer encapsulation efficiencies of at least 75 % by weight in relation to the initial amount of GABA in a solution to be encapsulated, even of more than 95-99% by weight. These high encapsulation efficiencies were, in addition, obtained by the application of encapsulating methods not proposed for in-water soluble substances.

Altogether, the specific design and features of the microcapsules of the invention improve ease of manufacture of the desired final product, providing at the same time an ideal microenvironment for preserving GABA throughout manufacture, storage, and passage through the gastrointestinal tract (or residence time in adverse environmental conditions) until arriving at their site of action, mainly in small intestine.

### BRIEF DESCRIPTION OF THE DRAWINGS

Figure 1 is a picture illustrating with bars the evolution of concentration of remaining GABA (in %) in the gastrointestinal environment for (A) non-encapsulated GABA and (B) encapsulated GABA with an alginate coating layer at different times (X-axis: 0, 60, 120, 180 minutes).
Figure 2 shows a picture taken with microscope Axioskop 40 (Zeiss) with dried microcapsules with probiotic bacteria and GABA surrounded by an alginate layer during the gastric simulation assay.

### DETAILED DESCRIPTION OF THE INVENTION

References to the methods of treatment by therapy or surgery or in vivo diagnosis methods in this description are to be interpreted as references to compounds, pharmaceutical compositions and medicaments of the present invention for use in those methods.

The present invention refers to multilayer microcapsules containing GABA. The term "microcapsule" is understood as a microparticle of size comprised from 10 to 3000 µm containing active components which is designed to release its components in the site of action. Preferably, the microcapsules have a size comprised from 50 to 1000 µm, more preferably from 50 to 500 µm, still more preferably from 100 to 400 µm.

The microcapsules of the invention may have different shapes and sizes, for example spheres, rods, cubes, tubes and hemispheres. Usually, the microcapsules are spherical or quasi-spherical. As used herein, the term "size" refers to a characteristic physical dimension. For example, in the most usuall case of a microcapsule that is spherical or substantially spherical, the size of the microcapsule corresponds to the diameter of the sphere or the equivalent diameter of the quasi-sphere assimilated as a sphere. The state of the art provides appropriate methods to calculate the diameter of microparticles that are substantially spherical. For example, the diameter of the microcapsules may be calculated using software such as Ellix Image Analysis Software (Microvision, France). When referring to a set of microcapsules as being of a particular size, it is contemplated that the set of microcapsules can have a distribution of sizes around the specified size. Thus, as used herein, a size of a set of microcapsules can refer to a mode of a distribution of sizes, such as a peak size of the distribution of sizes.

The term "GABA" as abbreviation of the γ-aminobutyric acid, or gamma-aminobutyric acid, refers to the compound as such in the acid form with the CAS number 56-12-2. Although data are depicted with GABA, pharmaceutically acceptable salts of the acid, or esters or other derivatives or analogues thereof with known or equivalent activity are also encompassed under this term, including among the derivatives or analogues phenibut (γ-amino-β-phenylbutyric acid), gamma-hydroxybuturic acid, pregalin (CAS 148553-50-8), gabapentin (CAS 60142-96-3), putrescine (CAS 110-60-1), gamma-Glutamyl-putrescine, gamma-Glutamyl-gamma-aminobutyraldehide, gamma-Glutamyl-gamma-aminobutyric acid. Examples of pharmaceutically acceptable salt sof GABA or of its derivatives include alkali metal salts such as sodium and potassium salts, alkaline earth metal salts such as calcium, magnesium and barium salts, an ammonium salt, and organic amine salts such as trimethylamine and triethylamine salts.

In a particular embodiment of the first aspect, it comprises alginate as the only hydrogel forming polymer and has such a molecular weight that gives a viscosity from 100 mPa.s to lower than 400 mPa.s, when measured at 25 °C in a dynamic viscosimeter and in a water solution at a concentration from 10 to 20 g/L of the said alginate polymer.

When in this description it is indicated that a composition has a particular viscosity within a range, it is related to the dynamic viscosity. Thus, the dynamic viscosity, at room temperature (i.e. 20 ±0.1 °C - 25 ±0.1 °C) and normal atmospheric pressure.

According to the European pharmacopoeia (8th edition, 2.2.8 "Viscosity"), dynamic viscosity or viscosity coefficient η is the tangencial force per unit surface, known as shearing stress T and expressed in pascals, necessary to move, parallel to the sliding plane, a layer of liquid of 1 square meter at a rate (v) of 1 meter per second relative to a parallel layer at a distance (x) of 1 meter. The ratio dv/dx is a speed gradient giving the rate of shear D expressed in reciprocal seconds (s⁻¹), so that η=T/D. The unit of dynamic viscosity is the pascal second (Pa.s ).

In another particular embodiment of the microcapsule of the first aspect, the alginate polymer is that having a molecular weight that when dissolved in a water solution in a concentration of 10 to 20 g/l, the solution has a viscosity from 200 mPa.s to 300 mPa.s, measured with at 25 °C in a dynamic viscosimeter. In a more particular embodiment, the alginate polymer is that with a molecular weight giving a viscosity selected from 200, 210, 220, 230, 240, 240, 260, 270, 280, 290, and 300 mPa.s measured at the previous indicated conditions (i.e., concentration, T^{a} and viscosimeter).

As indicated, a second aspect of the invention is a multilayer microcapsule, comprising:
(a) a core comprising the microcapsule as defined in the first aspect; and
(b) a coating layer surrounding the core, and comprising a hydrogel forming polymer.

In a particular embodiment of this second aspect, the core in the multilayer microcapsule consists of the microcapsule of the first aspect.

All the particular embodiments of the microcapsule of the first aspect apply to the multilayer microcapsule of the second aspect.

In another particular embodiment of the multilayer microcapsule of the second aspect, the hydrogel forming polymer is a polysaccharide selected from alginate and pectin.

The term "hydrogel forming polymer" relates to a network of crosslinked polymer chains that are hydrophilic, and sometimes found as a colloid in which water is the dispersion medium. A three-dimensional solid results from the hydrophilic polymer chains being held together by cross-links. The crosslinks which bond the polymers of a hydrogel fall under two general categories: physical and chemical. Physical crosslinks consist of hydrogen bonds, hydrophobic interactions, and chain entanglements (among others). Because of the inherent cross-links, the structural integrity of the hydrogel network does not dissolve from the high concentration of water. Hydrogels are highly absorbent (they can contain over 90% water) natural or synthetic polymeric networks.

The encapsulation efficiency near 100 % is further assured if, in addition, the multilayer microcapsule comprises, in another particular embodiment, one or more outer layers surrounding the coating layer (b), and comprising a polymer selected from alginate and pectin.

In another particular embodiment of the multilayer microcapsule of the second aspect, the coating layer (b) comprises alginate. More in particular, the coating layer (b) consists of alginate. The alginate is one that has a molecular weight that gives a viscosity from 100 mPa.s to lower than 400 mPa.s, when measured at 25 °C in a dynamic viscosimeter and in a water solution at a concentration from 10 to 20 g/L of the said alginate polymer.

In also another particular embodiment, the multilayer microcapsule comprises pectin in the coating layer (b). More in particular, the coating layer (b) consists of pectin.

In any of the microcapsules of the first and second aspects, they comprise in a particular embodiment, at least one probiotic, and/or at least one postbiotic. In another particular embodiment they comprise a prebiotic.

Thus, in particular, a part of GABA, embedded within the alginate and/or the pectin, or in the core (a) of the multilayer microcapsule, other active - components are contained in the microcapsules of the invention, said active-components selected from probiotics, postbiotics, mixtures of several probiotics, mixtures of several postbiotics or mixtures of probiotics and postbiotics.

This at least one probiotic, and/or at least one postbiotic or prebiotic are embedded with the GABA in the microcapsule of the first aspect. In the multilayer microcapsule of the second aspect, this at least one probiotic, and/or at least one postbiotic or prebiotic are in the core (a), and/or in the outer layer (b), and/or in any subsequent outer layer covering the outer layer (b).

All these active-components, i.e. GABA and if present any probiotic and/or postbiotic, and/or any prebiotic if desired, are "embedded" in the polymeric material. By "embedded" it is understood that the active components are distributed within a polymeric matrix. The term "entrapped" may also be employed with the same meaning. These active components are, in particular embodiments, located in separated spaces inside the multilayer microcapsule, which avoids negative interactions between incompatible active components, in particular, during prolonged shelf life. By "incompatible" components it is understood components that negatively affect the normal activity or structure of other components, thus decreasing their viability, bioavailability or functionality. For example, two probiotics may be incompatible between them because when found together one of them reduces the viability of the other.

GABA is mainly absorbed in small intestine but also all along intestinal tissue. On the other side, the more common place of action of probiotics and/or prebiotics and/or postbiotics are liberated is often the colon. As such, it is necessary that these active components be protected from the adverse conditions present in the gastrointestinal tract, mainly from gastric juices, and that they are progressively liberated along the intestines. The active components must also be protected from adverse conditions frequent in food or nutraceutical processing and during storage. Altogether the microcapsules of the invention possess (gastric) resistance, thermal resistance and mechanical resistance. "Gastric resistance" is understood as protection from conditions in the gastric juices in the stomach. Then, the microcapsules with GABA and if present any other postbiotic and/or probiotic, reach intact the intestine, and it is throughout the intestine transit that liberation takes place.

In another particular embodiment of either the microcapsules of the first or second aspects, this at least one probiotic is selected from the group consisting of lactic acid bacteria and bifidobacteria.

In particular, bacterial strains with well-known probiotic activity are selected from species of *Lactobacillus plantarum, Lactobacillus acidophilus, Lactobacillus casei, Bididobacterium bifidum, Lactobacillus gasseri, Lactobacillus helveticus.*

Postbiotics, also known as metabiotics or pharmacobiotics or heat-killed probiotic, are bioactive compounds produced by the metabolism of probiotics, mainly LAB. Postbiotics are also considered several compounds found inside probiotics that can be released to the environment before death. Enzymes, polysaccharides, organic acids, short-chain fatty acids (SCFA), cell surface proteins, vitamins, or lipids, are some of these metabolic products. All these metabolites called "postbiotics" have a functional effect on the microbiota, capable of modulating human health. GABA is, indeed one postbiotic.

Prebiotic active components are usually fermentable substances such as dietary fibres (see Diez-Gutiérrez, Lucía et al. 2020. "Gamma-Aminobutyric Acid and Probiotics: Multiple Health Benefits and Their Future in the Global Functional Food and Nutraceuticals Market." Journal of Functional Foods 64: 103669. https://www.sciencedirect.com/science/article/abs/pii/S1756464619305936 (January 16, 2020). Non limiting examples of prebiotics for use in the present invention are pectin, resistant starch, some polyphenols, beta-glucans, xylooligosccharides, galactooligosaccharides, oligofructose and inulin. In particular embodiments, the prebiotics are short-chain polysaccharides (oligosaccharides), which have demonstrated to be particularly effective in promoting growth of probiotic bacteria.

In also another particular embodiment of the first and second aspects, the microcapsule, or the multilayer microcapsule have a size comprised from 50 to 500 µm. In a more particular embodiment, the microcapsules have a size comprised from 100 to 400 µm. Even in a more particular embodiment, a size selected from 100, 150, 200, 250, 300, 350, and 400 µm.

Also provided is a process for the preparation of a microcapsule as defined in the first or second aspects or in any of its embodiments.

As indicated, the third aspect of the invention is a process for the preparation of a microcapsule as defined in the first aspect, and comprises:
i. preparing a solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3 of GABA:hydrogel forming polymer, wherein when said hydrogel forming polymer is alginate, it has such a molecular weight that gives a viscosity from 100 mPa.s to lower than 400 mPa.s, when measured at 25 °C in a dynamic viscosimeter and in a water solution at a concentration from 10 to 20 g/L;
ii. extruding the solution of step (i) through a nozzle with a section from 50 to 500 µm while applying a frequency of vibration to the nozzle from 100 Hz to 1000 Hz to obtain droplets; and
iii. harvesting the droplets into a divalent ion solution for hardening; and
iv. optionally drying the microcapsules obtained in step (iii).

In a particular embodiment of the process, in step (i) the ratio of GABA:hydrogel forming polymer is from 1:1.5 to 1:2.5. In a preferred embodiment, it is selected from 1:1.5, 1:1.75, 1:2.0, 1:2.25 and 1:2.5.

In a particular embodiment of the process, step (ii) of extruding while applying frequency is carried out by the technique known as prilling by vibration through one or more nozzles (i.e., through concentric nozzles), explained in more detail below.

In another particular embodiment of the process, the frequency of vibration is from 100 Hz to 500 Hz, more in particular from 100 Hz to 300 Hz. Even more in particular it is a frequency of vibration selected from 100, 150, 200, 250, 300 Hz.

In another particular embodiment, step (iii) of harvesting is performed from 1 to 10 minutes, more in particular from 2 to 5 minutes, even more in particular for a time selected from 2, 3, 4, and 5 minutes.

The solutions are extruded through nozzles (or concentric nozzles in the multilayer microcapsules) into a divalent ion solution for hardening. The divalent ion is selected from copper, barium, strontium, calcium, zinc, cobalt and nickel. In preferred embodiments, the divalent ion is calcium. In particular embodiments, the solution is an aqueous calcium salt solution with concentration comprised from 1 to 20% (w/v), preferably from 1 to 15%, more preferably from 1 to 10. In some embodiments the calcium salt solution has a concentration comprised from 1 to 3%. This concentration is suitable for microcapsules containing alginate as sole polymer. In other embodiments the calcium salt solution has a concentration comprised from 7 to 15%. This concentration is suitable for microcapsules also containing pectin. In preferred embodiments the calcium salt is calcium chloride.

In another particular embodiment, step (iv) is present. The microcapsules are dried to a water activity value lower than 0.4, preferably lower than 0.3. Drying may be achieved by conventional methods but is preferentially done at mild temperatures. Non-limiting examples of appropriate drying methods are conventional ovens, preferentially working at temperatures comprised from 40 to 60 °C or by fluid bed drying, preferentially working at temperatures comprised from 20 to 60 °C, and lyophilisation, preferably at temperatures below 0 °C. When lyophilisation is the selected drying method a cryoprotectant may be added. Suitable cryoprotectants for use in food products and nutraceuticals are known in the art. In a particular embodiment drying is achieved by fluid bed drying, preferentially working at temperatures comprised from 20 to 60 °C.

The fourth aspect relates to the process for the preparation of a multilayer microcapsule as defined in the second aspect, and it comprises:
(i) preparing a core (a) solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3 of GABA:hydrogel forming polymer, wherein when said hydrogel forming polymer is alginate it has such a molecular weight that gives a viscosity from 100 mPa.s to lower than 400 mPa.s, when measured at 25 °C in a dynamic viscosimeter and in a water solution at a concentration from 10 to 20 g/L of the said alginate polymer;
(ii) preparing a coating layer (b) solution comprising a hydrogel forming polymer;
(iii) extruding the core solution (a) and the coating layer (b) of steps (i) and (ii) through concentric nozzles, each with a section from 50 to 500 µm and having different section or diameters defining an inner nozzle and an external nozzle, while applying a frequency of vibration from 100 Hz to 1000 Hz to the nozzles to obtain multilayer droplets, in such a way that the core solution flows through the inner nozzle and the coating layer solution flows through the external nozzle;
(iv) harvesting the multilayer droplets into a divalent ion solution for hardening; and
(v) optionally drying the microcapsules obtained in step (iv).

All the previous particular embodiments of the process of the third aspect do also apply to the process of the fourth aspect.

In a particular embodiment of the fourth aspect, the hydrogel forming polymer of the coating layer (b) solution comprises alginate or pectin, and optionally at least one probiotic, and/or at least one postbiotic.

In even another particular embodiment, the process of the fourth aspect comprises preparing and applying also by the use of multiple concentric nozzles additional outer layers, for example of a hydrophobic material having a melting point comprised from 35 to 90 °C, to obtain multilayer capsules of more than two layers or levels.

In particular, the outer layer confers enhanced resistance to mechanical and thermal stresses, which are typical of the food manufacturing processes. Said outer layer additionally provides an effective barrier to moisture. The moisture barrier is a great advantage since it prevents leakage of soluble postbiotic components, but also because it avoids an increase of water activity inside the microcapsule, which would be damaging to the entrapped probiotics.

In a particular embodiment of any of the processes of the third and fourth aspects, the polymer concentration in the solutions (a) and (b) is from 1 to 3 % (w/v) when the polymer is alginate and from 3 to 7% (w/v) when the polymer is pectin. "% w/v" is understood as per cent mass concentration, i.e., per cent mass/volume. Preferably the alginate solution comprises from 1.5 to 2% (w/v) alginate and the pectin solution comprises from 4 to 6 (w/v) pectin, since theses concentrations have been found to be the optimal for the invention because they combine an appropriate matrix for the microcapsule layers as well as appropriate viscosity for the preparation process.

Also, in another particular embodiment of any of the processes of the third and fourth aspects, when a probiotic and/or postbiotic is present, it is embedded with the GABA in the hydrogel forming polymer of the monolayer microcapsule version, or in the core (a) and/or coating layer (b) of a multilayer microcapsule, and at a concentration comprised from 10⁷ to 10⁹ cfu/mL solution. The postbiotic, when it is present is at a concentration comprised from 0.01 to 0.05 g/mL solution, and also embedded with the GABA in the hydrogel forming polymer of the monolayer microcapsule version, or in the core (a) and/or coating layer (b) of a multilayer microcapsule.

Preferably, in the process for the preparation of multilayer microcapsules, the polymer embedding GABA in the core solution is alginate having a molecular weight that gives a viscosity from 100 mPa.s to lower than 400 mPa.s, said viscosity when measured at 25 °C in a dynamic viscosimeter and in a water solution at a concentration from 10 to 20 g/L. In particular embodiments, the polymer in the core solution, and also in the coating solution is alginate with the molecular weight previously disclosed.

In another embodiment of the process for the preparation of multilayer microcapsules, the polymer embedding GABA in the core solution is alginate having a molecular weight that gives a viscosity from 100 mPa.s to lower than 400 mPa.s, when measured at 25 °C in a dynamic viscosimeter and in a water solution at a concentration from 10 to 20 g/L of the said alginate polymer; and the coating layer solution comprises pectin.

Also, in another particular embodiment of any of the processes of the third and fourth aspects, it additionally comprises the step of applying an electrostatic force to the extruding microcapsules.

By "prilling by vibration through a nozzle or concentric nozzles" it is understood simultaneously extruding the solutions through vibrating nozzles or concentric nozzles. As previously explained and as will be evident to the skilled person, the core solution is extruded through the nozzle of smaller diameter, while the inner layer solution is extruded through the nozzle of larger diameter.

In the prilling by vibration technique, the vibrating nozzles generate a sinusoidal wave in the extruded liquid jet, particularly in the laminar flow regime, to form droplets with a considerably reduced size distribution, with one droplet formed per hertz frequency applied. The size of the obtained microcapsules can be selected within a certain range depending on the nozzles diameter and the applied frequency. A compromise is required between the jet flow velocity and the frequency applied, because the optimal range of stable bead formation is limited by several factors: too small jet flow velocity resulting in no jet formation, but too large jet flow velocity resulting in a wavy jet; and too small frequencies resulting in satellite droplet formation, but too large vibration frequency resulting in an unstable droplet formation. In addition to the above, other parameters affect the characteristics of resulting capsules: the relative jet flow velocity (i.e., the relation between the jet velocities of the different solutions), the nozzle geometry, the relation between nozzle diameters or the difference in dynamic viscosity of the polymer solutions.

In order to obtain suitable results, the use of an outer nozzle diameter approximately double sizing the inner one is recommended. Preferably, the smaller nozzle has a diameter comprised from 100 to 200 µm, the medium nozzle has a diameter comprised from 200 to 400 µm and, if present, the larger nozzle has a diameter comprised from 300 to 600 µm.

Regarding other relevant parameters, the vibration frequency is comprised from 100 to 1000 Hz, preferably from 100 Hz to 500 Hz, more in particular from 100 Hz to 300 Hz. Even more in particular it is a frequency of vibration selected from 100, 150, 200, 250, 300 Hz, as previously indicated; and the relative jet flow velocity of the solutions being extruded through the nozzles is 1:1 to 1:2.5 (when extruding two solutions), more preferably from 1:1 to 1:1.5. The relative jet flow velocity is the relation between the jet velocities of the solutions being extruded through the smaller nozzle and, the larger nozzle and is expressed as jet flow velocity of smaller nozzle: jet flow velocity of larger nozzle.

In some embodiments the prilling by vibration is combined with addition of an electrostatic force to ionize the air through which the droplets form. This may be done by means of an electrostatic device and enables optimizing droplet formation, and thus microcapsule properties. Thus, in one embodiment the method additionally comprises the step of applying an electrostatic force to the extruding microcapsules. An electrostatic force from 300 to 700 mV is useful to work with medium-high viscous solutions (the optimal conditions depend on the dynamic viscosity of the solutions). The electrostatic force achieves narrow particle size distribution and enables a reduction of the microcapsule diameter of around 20-40 %, as well as a more spherical shape of the microcapsule and a better layer distribution. In a preferred embodiment an electrostatic force comprised from 300 to 2000 mV, in particular from 500 to 1000 mV, and more in particular from selected from 500, 550, 600, 650, 700, 750, 800, 850, 900, 850 and 1000 mV, is applied.

When the microcapsule of the invention contains one or more outer layers, the preparation process may comprise applying them over a first dry capsule comprising a core (a) and a coating layer (b) prepared by prilling by vibration as defined above. Applying of the outer layer(s) in this case may be done by conventional methods such as pan coating, atomic layer deposition or fluid bed coating, a part of using as explained, the multiple concentric nozzles in the prilling by vibration technique. In a particular embodiment, the one or more outer layers are applied by fluid bed coating. For example, the microcapsule outer layer could be applied in a Wurster fluid bed process where alginate solution is atomized from a bottom-spray nozzle at air inlet temperature is selected to dry the deposited layer. If further outer layers are desired, said layers are also preferably applied by fluid bed coating in continuous process changing feed solution.

As a result of the above processes, microcapsules are obtained that contain or retain high amounts of GABA. These microcapsules are ideal for applications where it is important to efficiently provide GABA in intestine, where it is absorbed. They multilayer microcapsules also ideal to avoid negative interactions between different active components, for example if probiotics and/or postbiotics and/or prebiotics are present that are incompatible with GABA or with each other. All microcapsules of the invention possess high processing and enteric or environmental resistance.

Microcapsule characterization may be done using several techniques. As already mentioned, microscope (Zeiss) and Ellix Image Analysis Software (Microvision Image Analysis) can be used to feature size and shape analysis. The bacteria concentration per gram of final product can be calculated by breaking the microcapsules with a solution of 0.1 M of sodium citrate. Once the microcapsules are completely broken, serial dilutions are made and poured in MRS agar plates in order to count the viable bacteria contained in the microcapsules. Moreover, microcapsules can be faced to gastric and intestinal conditions to prove their resistance and their ability to reach the colon. The same citrate solution can be used to break the microcapsules and to determine the postbiotic and/or prebiotic concentration by chromatographic techniques, for example.

The microcapsules of the invention are very well adapted for the manufacture of functional food or feed products, as indicated. These microcapsules of the particular embodiments comprising probiotics and/or postbiotics a part of providing high amounts of GABA, further imply the advantage of providing known dosages of these active-components, which is one of the handicaps when different probiotic strains are added separately, mainly if some of them are incompatible with each other.

In one embodiment, the microcapsules of the invention to be added in the final food product contain from 10⁵ to 10¹⁰ cfu of each probiotic strain per g (dry) microcapsules and/or from 0.1 to 0.5 g of prebiotic and/or postbiotic per g (dry) microcapsules. In particular embodiments the amount of each probiotic strain in the microcapsule is from 10⁷ to 10⁹ cfu of each probiotic strain per g (dry) microcapsules and/or from 0.2 to 0.4 g prebiotic and/or postbiotic per g (dry) microcapsules. Preferably, the amount of each probiotic strain in the microcapsule is from 10⁸ to 10⁹ cfu of each probiotic strain per g (dry) microcapsules and/or from 0.2 to 0.3 g prebiotic and/or postbiotic per g (dry) microcapsules. "cfu" means colony forming units.

The food or feed products containing the microcapsules of the invention may be any type of functional food or feed, such as dairy products (fermented milk, yogurt, kefir, ice-cream, cheese, etc.), meat-based products (like sausages, meat pies, etc.), soy-bean based products (like tofu, miso, yakult, etc.), infant food (formula milk, cereals, etc.), formula food for special medical purposes, nutrition bars, snack bars, breakfast cereal and cereal-based products, confectionary products, desserts, fruit juices, cereal-based juices, vegetable juices, mayonnaise, etc. The microcapsules of the invention may also be directly marketed as a food ingredient, with or without further appropriate additives or in combination with other compounds, to be used to prepare a food or feed product. The microcapsules of the invention are however also useful for the preparation of nutraceuticals, understanding the term "nutraceutical" as a product derived from a food that is marketed in the form of medicine and is demonstrated to have a physiologic benefit or to provide protection against chronic disease. Dietary supplements are a typical example for nutraceuticals. Usually, nutraceuticals take the form of pills, tablets, capsules, ovules, gels, granulates.

The microcapsules of the invention are also ingredients in pharmaceutical and/or cosmetic compositions, mainly in form of pills, tablets, capsules, ovules, gels, or granulates. Thus, the invention also relates to pharmaceutical and/or cosmetic compositions comprising a therapeutically or cosmetic effective amount of the microcapsules of the first and second aspect. The pharmaceutical compositions comprising a therapeutically effective amount of the microcapsules of the first and second aspect, do also contain the pharmaceutically or cosmetically acceptable excipients and/or carriers known by the skilled person in the art. These pharmaceutical compositions are for use in the in treatment and/or prevention of all those medical indications in which GABA, its pharmaceutically acceptable salts, or any derivative is used. More in particular, the pharmaceutical compositions comprising a pharmaceutically effective amount the microcapsules of the first and second aspect, are for use in the prevention and/or treatment of sleep disorders or temporal and spatial memory, epilepsy, depression, diabetes, asthmatic disorders, cancer, neural diseases such as schizophrenia, Alzheimer's disease, Parkinson's disease or Huntington's disease.

The term "pharmaceutically or therapeutically effective amount" as used herein, means an amount of an active agent high enough to deliver the desired benefit, but low enough to avoid serious side effects within the scope of medical or veterinary judgment.

The term "pharmaceutically acceptable" as used herein pertains to compounds, materials, compositions, and/or dosage forms which are, within the scope of sound medical and veterinary judgment, suitable for use in contact with the tissues of a subject (e.g., human or any other animal) without significant toxicity, irritation, allergic response, or other problem or complication, commensurate with a reasonable benefit/risk ratio. Each carrier, excipient, etc., must also be "acceptable" in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Suitable carriers, excipients, etc. can be found in standard pharmaceutical texts, and include, as a way of example preservatives, pH adjusting agents, tonicity agents to adjust osmolality, chelating agents, penetration enhancers, bioadhesive polymers, stabilizers, viscosizing agents, and antioxidants.

The term "cosmetically acceptable" or "dermatological acceptable" which is herein used interchangeably refers to that excipients or carriers suitable for use without undue toxicity, incompatibility, instability, allergic response, among others.

The term "safe and effective amounts" is defined as any amount sufficient to significantly improving the cosmetic appearance of the body, such as skin, without substantial irritation, but low enough to avoid serious side effects (at a reasonable benefit/risk ratio), within the scope of sound medical judgement.

Throughout the description and claims the word "comprise" and variations of the word, are not intended to exclude other technical features, additives, components, or steps. Furthermore, the word "comprise" encompasses the case of "consisting of". The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### Example A: effect of encapsulating alginate in GABA retention

An appropriate amount of GABA (56-12-2) was disolved in a solution of alginate 15 or 20 g/L prepared in deionized water at room temprerature under gentle stirring. These solutions were extruded through a 150 (µm) micron nozzle using a Encapsulator B-395 Pro (Büchi) adjusting the jet flow velocity 3 mL/min. The jet was broken down into small droplets applying a frequency of 500 Hz. The obtained droplets were harvested in a Calcium Chloride bath (0.1M), as the solution of divalent ion solution for hardening. A volumen of 50 mL of encapsulating solution is collected in a 100 mL bathafter 1 minute for beads hardening.

In order to compare both experimental results (15 or 20 g/L), GABA concentration selected was definied as a half of alginate material dissolved.

The obtained microcapsules were characterized according to their bead diameter (optical microscope observation), size distribution (image analysis software) and GABA retained (UPLC-MS).

The encapsulation efficiency (EE) was measured as the ratio between the GABA encapsulated and the GABA dissolved and extruded (i.e. initially added GABA). Any of the techniques for detecting GABA known by the skilled person in the art can be used to determine this EE, meanwhile the same technique be used for the taking the two measures (encapsulated GABA and dissolved and extruded GABA).

Using a solution 20 g/L alginate with high molecular weight and viscosity around 700- 800 mPa s, the EE measured is arround the 30% after 1 minute of hardening (see data in next Example B). A high porosity of the alginate matrix was postulated as the cause of a low retention of this hydrosoluble molecule due to a diffusion phenomenon.

A experimental design was defined in order to improvethe GABA retention. The experimental factors selected were the ratio GABA:alginate (weight) as well as the viscosity of alginate solution. The optimum result was achieved with a ratio 1:2, with a low-medium viscosity, around 170-230 mPa.s (20 g/L solution at 25°C) (See data in next Example B).

With these factors, an alginate retention greater than 75% was achieved after 1 minute of hardening. The effective payload of dried beads could be greater than 25%.

The GABA evolution in a aqueous bath showed a retention greater than 50% after 5 minutes of non-dried beads suspension in a stirring bath.

### Example B: influence of the probiotic co-encapsulation in GABA retention

GABA is produced by a probiotic bacteria strain so it could be interesting to achieve the co-encapsulation of bacteria and GABA in a unique structure.

However, the inclusion of bacteria into the encapsulating matrix has to be evaluated to verify that achieved GABA retention is not affected.

The optimum result from the experimental design conducted in the example A (an alginate 20 g/L solution, 170-230 mPa.s at 25°C; and a ratio 1:2 for GABA and alginate) is modified with the inclusion of a strain concentration 10⁵ - 10⁶ UFC/L.

The results are showed on table 1. The presence of probiotic bacteria inside the microcapsule is not a problem for the GABA retention: there is even a benefit in short-term retention.

Moreover, the probiotic bacteria showed a very good stability during process and shelf life, maintaining their viability up to 10⁸ UFC/g of final product.

**Table 1: GABA retained during hardening step from encapsulation process**

| | 1 min | 5 min |
|---|---|---|
| Monolayer HV (Comparative) | 38 | 30 |
| Monolayer LV (170-230 mPa.s) | 75 | 50 |
| Monolayer LV + bacteria | 65 | 50 |

| | | |
|---|---|---|
| - HV means high viscosity (i.e., an alginate polymer with a molecular weight giving a dynamic viscosity of 700-800 mPa.s, at 25 °C and at a concentration of 20 g/L of the alginate polymer) - LV means low viscosity (i.e., an alginate polymer with a molecular weight giving a dynamic viscosity of 170-230 mPa.s, at 25 °C and at a concentration of 20 g/L of the alginate polymer) | | |

### Example C: encapsulation of GABA non purified from fermentation medium

The process required for GABA purification is not always possible or interesting. The encapsulaciton system was tested to retain GABA from a fermentation medium (conditioned media) after bacteria removal.

Bacteria were removed from fermentation medium by centrifugation during 15min at 12000rpm. After that, the medium was filtered trough a polystyrene filter of 0.22 micron. Alginate was added into the medium maintaning the optimum conditions defined in the example A.

The EE was determined over 90% of GABA retention after 5 minutes of suspension in a water bath medium.

### Example D: influence of the application of a coating hydrogel layer in GABA retention

The appliction of a coating layer around a hydrogel bead was showed as a viable strategy to improve the active compounds retention and stabiliy because it could be an impediment for the active compound diffusion or a barrier for oxygen, light.

However, the application of a good hydrogel coating around the GABA bead had to consider the high water solubility of the active compound. It was necessary to solve with a new experimental design the optimum formulation of the coating to minimize the diffusion pathway of GABA.

Adjusting the characteristics of encapsulating solution for the external layer, such as alginate concentration and molecular weight distribution of polymer chains, it was possible to increase the GABA retention up to 90%.

Moreover, an external layer can be formulated with a different material. A new experiment was conducted applying a 60 g/L of a pectin solution through concentric nozzles with a diameter (or section) of 150 µm the diameter of the inner nozzle, and of 300 µm the diameter of the external nozzle through which the pectine solution was extruded. A total retention of GABA was observed in the same observation time.

The barrier layer to control GABA diffusion can be applied even if the microcapsule is based on a co-encapsulation process (Example B).

### Example E: gastric resistance of the encapsulated GABA

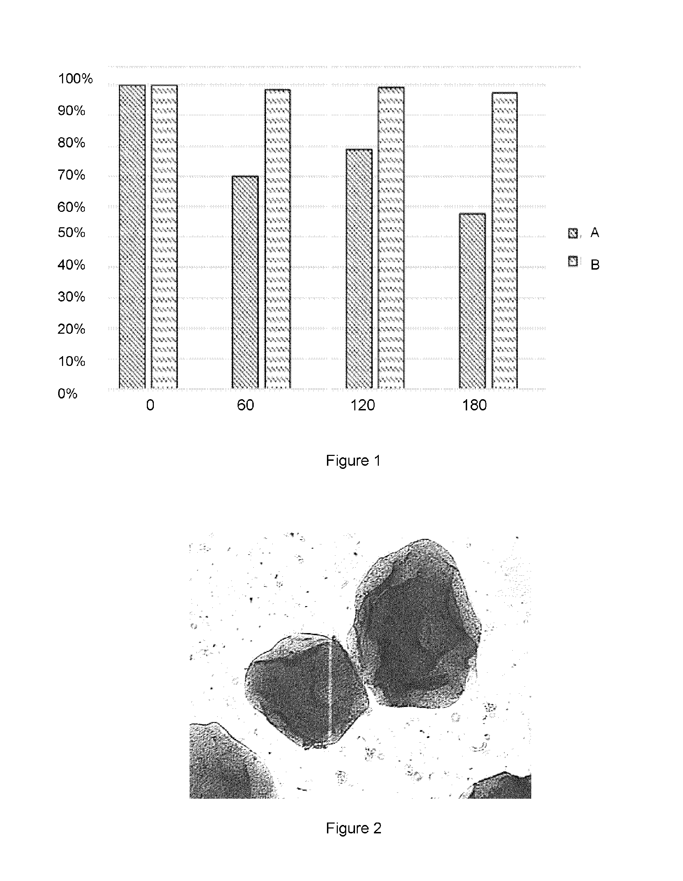

According with the Chávarri, M. et al. 2010 description (DOI: 10.1016/j.ijfoodmicro.2010.06.022), the GABA stability has been evaluated in a gastrointestinal simulation assay. Comparing the stability of non-encapsulated and encapsulated GABA, it is possible to conclude the improvement of molecule stability by encapsulation in an alginate coated structure (Figure 1).

Moreover, an expected result from an alginate microcapsule is the improvement of bacteria survival in a gastrointestinal simulation assay: Variability on encapsulated bacteria viability before and after the gastrointestinal simulation assay is lower than 0,5 log UFC/g. Figure 2, which is a microscope image Axioskop 40 (Zeiss) illustrates the integrity of microcapsule structure at the end of gastric simulation assay.

### REFERENCES CITED IN THE APPLICATION

- CN102125168 (HANGZHOU KANGDEQUAN FEED CO LTD)
- JP2017073991 (AOMORI PREFECTURAL IND TECH RES CENTER CALORIA JAPAN CO LTD)
- Pandey, P. and Mishra, H.N. (2020). Co-microencapsulation of γ-aminobutyric acid (GABA) and probiotic bacteria in thermostable and biocompatible exopolysaccharide matrix. LWT-Food Science and Technology 136 (2021) 110293.
- Polkovnikova, Y. and Korionova, K. (2019), Microcapsules made of sodium alginate for the prolonged release of phenibut, J Res Pharm; vol. no. 23(6), pp.:1040-1047
- EP3205216 (FUNDACIÓN TECNALIA RESEARCH & INNOVATION)
- Diez-Gutiérrez, Lucía et al. 2020. "Gamma-Aminobutyric Acid and Probiotics: Multiple Health Benefits and Their Future in the Global Functional Food and Nutraceuticals Market." Journal of Functional Foods 64: 103669. https://www.sciencedirect.com/science/article/abs/pii/S1756464619305 936 (January 16, 2020)
- Chávarri, M. et al. 2010 description (DOI: 10.1016/j.ijfoodmicro.2010.06.022

## Claims

1. A microcapsule comprising:
gamma-aminobutyric acid (GABA) embedded in a hydrogel forming polymer selected from alginate polymer, or a mixture of alginate and pectin, wherein the alginate has such a molecular weight that gives a viscosity from 100 mPa.s to lower than 400 mPa.s, when measured at 25 °C in a dynamic viscosimeter and in a water solution at a concentration from 10 to 20 g/L of the said alginate polymer.

2. The microcapsule according to claim 1, which comprises alginate as the only hydrogel forming polymer and has such a molecular weight that gives a viscosity is from 100 mPa.s to lower than 400 mPa.s, when measured at 25 °C in a dynamic viscosimeter and in a water solution at a concentration from 10 to 20 g/L of the said alginate polymer.

3. A multilayer microcapsule, comprising:
(a) a core comprising the microcapsule as defined in any one of claims 1-2; and
(b) a coating layer surrounding the core, and comprising a hydrogel forming polymer.

4. The multilayer microcapsule according to claim 3, wherein the hydrogel forming polymer is a polysaccharide selected from alginate and pectin.

5. The multilayer microcapsule according to any one of claims 3-4, that comprises one or more outer layers surrounding the coating layer (b), and comprising a polymer selected from alginate and pectin.

6. The microcapsule according to any one of the claims 3-5, wherein the coating layer (b) comprises alginate.

7. The multilayer microcapsule according to any of the claims 3-6, wherein the coating layer (b) comprises pectin.

8. The microcapsule according to any one of the claims 1-2, or the multilayer microcapsule according to any one of the claims 3-7, which further comprises at least one probiotic, and/or at least one postbiotic.

9. The microcapsule according to any one of claims 1-2, or the multilayer microcapsule according to any one of the claims 3-7, which has a size comprised from 50 to 500 µm.

10. A process for the preparation of a microcapsule as defined in any one of the claims 1-2, or as defined in claim 8, comprising:
i. preparing a solution comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3.0 of GABA:hydrogel forming polymer, wherein when said hydrogel forming polymer is alginate it has such a molecular weight that gives a viscosity from 100 mPa.s to lower than 400 mPa.s, when measured at 25 °C in a dynamic viscosimeter and in a water solution at a concentration from 10 to 20 g/L of the said alginate polymer;
ii. extruding the solution of step (i) through a nozzle with a section from 50 to 500 µm, while applying a frequency of vibration to nozzles from 100 Hz to 1000 Hz to obtain droplets; and
iii. harvesting the droplets into a divalent ion solution for hardening; and
iv. optionally drying the microcapsules obtained in step (iii)

11. A process for the preparation of a multilayer microcapsule as defined in any one of the claims 3-8 comprising:
(i) preparing a core solution (a) comprising gamma-aminobutyric acid (GABA), and optionally at least one probiotic, and/or at least one postbiotic, embedded in a hydrogel forming polymer selected from alginate and/or pectin, by dissolving while stirring the GABA in a solution comprising the hydrogel forming polymer in a weight ratio from 1:1 to 1:3 of GABA:hydrogel forming polymer, wherein when said hydrogel forming polymer is alginate it has such a molecular weight that gives a viscosity from 100 mPa.s to lower than 400 mPa.s, when measured at 25 °C in a dynamic viscosimeter and in a water solution at a concentration from 10 to 20 g/L of the said alginate polymer;
(ii) preparing a coating layer (b) solution comprising a hydrogel forming polymer,
(iii) extruding the core solution (a) and the coating layer (b) solution of steps (i) and (ii) through concentric nozzles, each with a section from 50 to 500 µm and having different section or diameters defining an inner nozzle and an external nozzle, while applying a frequency vibration from 100 Hz to 1000 Hz to the nozzles to obtain multilayer droplets, in such a way that the core solution flows through the inner nozzle and the coating layer solution flows through the external nozzle; and
(iv) harvesting the multilayer droplets into a divalent ion solution for hardening; and
(v) optionally drying the microcapsules obtained in step (iv).

12. The process according to any one of claims 10-11, wherein the polymer concentration in the solutions (i) and (ii) is from 1 to 3 % (w/v) when the polymer is alginate and from 3 to 7% (w/v) when the polymer is pectin.

13. The process according to any of the claims 10-12, wherein the probiotic, when it is present, is at a concentration comprised from 10⁷ to 10⁹ cfu/mL solution and the postbiotic, when it is present, is at a concentration comprised from 0.01 to 0.05 g/mL solution.

14. A food or feed product, a food or feed ingredient, a nutraceutical, a pharmaceutical or a cosmetic composition comprising microcapsules as defined in any one of the claims 1-9.

## Patentansprüche

1. Eine Mikrokapsel, umfassend:
Gamma-Aminobuttersäure (GABA), die in ein Hydrogel bildendes Polymer eingebettet ist, das aus Alginatpolymer oder einer Mischung aus Alginat und Pektin ausgewählt ist, wobei das Alginat ein solches Molekulargewicht hat, das eine Viskosität von 100 mPa.s bis weniger als 400 mPa.s ergibt, wenn sie bei 25 °C in einem dynamischen Viskosimeter und in einer Wasserlösung bei einer Konzentration von 10 bis 20 g/L des Alginatpolymers gemessen wird.

2. Die Mikrokapsel nach Anspruch 1, welche Alginat als das einzige Hydrogel bildende Polymer umfasst und ein solches Molekulargewicht hat, dass eine Viskosität von 100 mPa.s bis weniger als 400 mPa.s ergibt, wenn sie bei 25 °C in einem dynamischen Viskosimeter und in einer Wasserlösung bei einer Konzentration von 10 bis 20 g/L des Alginatpolymers gemessen wird.

3. Eine mehrschichtige Mikrokapsel, umfassend:
(a) einen Kern, der die Mikrokapsel wie in einem der Ansprüche 1 bis 2 definiert umfasst; und
(b) eine Überzugsschicht, die den Kern umgibt und ein Hydrogel bildendes Polymer umfasst.

4. Die mehrschichtige Mikrokapsel nach Anspruch 3, wobei das Hydrogel bildende Polymer ein Polysaccharid ist, das aus Alginat und Pektin ausgewählt ist.

5. Die mehrschichtige Mikrokapsel nach einem der Ansprüche 3 bis 4, die eine oder mehrere äußere Schichten umfasst, die die Überzugsschicht (b) umgeben, und umfassend ein Polymer, das aus Alginat und Pektin ausgewählt ist.

6. Die Mikrokapsel nach einem der Ansprüche 3 bis 5, wobei die Überzugsschicht (b) Alginat umfasst.

7. Die mehrschichtige Mikrokapsel nach einem der Ansprüche 3 bis 6, wobei die Überzugsschicht (b) Pektin umfasst.

8. Die Mikrokapsel nach einem der Ansprüche 1 bis 2 oder die mehrschichtige Mikrokapsel nach einem der Ansprüche 3 bis 7, welche ferner mindestens ein Probiotikum und/oder mindestens ein Postbiotikum umfasst.

9. Die Mikrokapsel nach einem der Ansprüche 1 bis 2 oder die mehrschichtige Mikrokapsel nach einem der Ansprüche 3 bis 7, welche eine Größe von 50 bis 500 µm hat.

10. Ein Verfahren zur Herstellung einer Mikrokapsel wie in einem der Ansprüche 1 bis 2 definiert, oder wie in Anspruch 8 definiert, umfassend:
i. herstellen einer Lösung umfassend Gamma-Aminobuttersäure (GABA) und wahlweise mindestens ein Probiotikum und/oder mindestens ein Postbiotikum, eingebettet in ein Hydrogel bildendes Polymer, ausgewählt aus Alginat und/oder Pektin, durch Auflösen unter Rühren der GABA in einer Lösung umfassend das Hydrogel bildende Polymer in einem Gewichtsverhältnis von 1:1 bis 1:3,0 von GABA:Hydrogel bildendem Polymer, wobei das Hydrogel bildende Polymer, wenn es Alginat ist, ein solches Molekulargewicht hat, das eine Viskosität von 100 mPa.s bis weniger als 400 mPa.s ergibt, wenn sie bei 25 °C in einem dynamischen Viskosimeter und in einer Wasserlösung bei einer Konzentration von 10 bis 20 g/L des Alginatpolymers gemessen wird;
ii. extrudieren der Lösung aus Schritt (i) durch eine Düse mit einem Querschnitt von 50 bis 500 µm, während eine Vibrationsfrequenz auf Düsen von 100 Hz bis 1000 Hz angewendet wird, um Tröpfchen zu erhalten; und
iii. ernten der Tröpfchen in eine Lösung aus zweiwertigen Ionen zur Härtung; und
iv. wahlweise trocknen der in Schritt (iii) erhaltenen Mikrokapseln.

11. Ein Verfahren zur Herstellung einer mehrschichtigen Mikrokapsel wie in einem der Ansprüche 3 bis 8 definiert, umfassend:
(i) herstellen einer Kernlösung (a) umfassend Gamma-Aminobuttersäure (GABA) und wahlweise mindestens ein Probiotikum und/oder mindestens ein Postbiotikum, eingebettet in ein Hydrogel bildendes Polymer, ausgewählt aus Alginat und/oder Pektin, durch auflösen unter Rühren der GABA in einer Lösung umfassend das Hydrogel bildende Polymer in einem Gewichtsverhältnis von 1:1 bis 1:3 von GABA zum Hydrogel bildendem Polymer, wobei das Hydrogel bildende Polymer, wenn es Alginat ist, ein solches Molekulargewicht hat, das eine Viskosität von 100 mPa.s bis weniger als 400 mPa.s ergibt, wenn sie bei 25 °C in einem dynamischen Viskosimeter und in einer Wasserlösung bei einer Konzentration von 10 bis 20 g/L des Alginatpolymers gemessen wird;
(ii) herstellen einer Lösung aus Überzugsschicht (b) umfassend ein Hydrogel bildendes Polymer,
(iii) extrudieren der Kernlösung (a) und der Lösung aus Überzugsschicht (b) der Schritte (i) und (ii) durch konzentrische Düsen mit jeweils einem Querschnitt von 50 bis 500 µm und mit unterschiedlichen Querschnitten oder Durchmessern, die eine innere Düse und eine äußere Düse definieren, während eine Frequenzschwingung von 100 Hz bis 1000 Hz auf die Düsen angewendet wird, um mehrschichtige Tröpfchen zu erhalten, so dass die Kernlösung durch die innere Düse fließt und die Lösung aus Überzugsschicht durch die äußere Düse fließt; und
(iv) ernten der mehrschichtigen Tröpfchen in eine Lösung aus zweiwertigen Ionen zur Härtung; und
(v) wahlweise Trocknen der in Schritt (iv) erhaltenen Mikrokapseln.

12. Das Verfahren nach einem der Ansprüche 10 bis 11, wobei die Polymerkonzentration in den Lösungen (i) und (ii) von 1 bis 3 % (w/v) reicht, wenn das Polymer Alginat ist, und von 3 bis 7 % (w/v) reicht, wenn das Polymer Pektin ist.

13. Das Verfahren nach einem der Ansprüche 10 bis 12, wobei das Probiotikum, wenn es vorhanden ist, in einer Konzentration von 10⁷ bis 10⁹ KBE/mL Lösung vorliegt und das Postbiotikum, wenn es vorhanden ist, in einer Konzentration von 0,01 bis 0,05 g/mL Lösung vorliegt.

14. Ein Nahrungs- oder Futtermittelprodukt, ein Nahrungs- oder Futtermittelzutat, eine nutrazeutische, eine pharmazeutische oder eine kosmetische Zusammensetzung, umfassend Mikrokapseln wie in einem der Ansprüche 1 bis 9 definiert.

## Revendications

1. Une microcapsule comprenant :
de l'acide gamma-aminobutyrique (GABA) incorporé dans un polymère formant un hydrogel choisi parmi un polymère d'alginate, ou un mélange d'alginate et de pectine, dans lequel l'alginate a un poids moléculaire tel qui donne une viscosité de 100 mPa.s à moins de 400 mPa.s, lorsqu'elle est mesurée à 25 °C dans un viscosimètre dynamique et dans une solution aqueuse à une concentration de 10 à 20 g/L dudit polymère d'alginate.

2. La microcapsule selon la revendication 1, qui comprend de l'alginate comme seul polymère formant un hydrogel et qui a un poids moléculaire tel qui donne une viscosité de 100 mPa.s à moins de 400 mPa.s, lorsqu'elle est mesurée à 25 °C dans un viscosimètre dynamique et dans une solution aqueuse à une concentration de 10 à 20 g/L dudit polymère d'alginate.

3. Une microcapsule multicouche, comprenant :
(a) un noyau comprenant la microcapsule telle que définie dans l'une quelconque des revendications 1 à 2 ; et
(b) une couche de revêtement entourant le noyau, et comprenant un polymère formant un hydrogel.

4. La microcapsule multicouche selon la revendication 3, dans laquelle le polymère formant un hydrogel est un polysaccharide choisi parmi l'alginate et la pectine.

5. La microcapsule multicouche selon l'une quelconque des revendications 3 à 4, qui comprend une ou plusieurs couches externes entourant la couche de revêtement (b), et comprenant un polymère choisi parmi l'alginate et la pectine.

6. La microcapsule selon l'une quelconque des revendications 3 à 5, dans laquelle la couche de revêtement (b) comprend de l'alginate.

7. La microcapsule multicouche selon l'une quelconque des revendications 3 à 6, dans laquelle la couche de revêtement (b) comprend de la pectine.

8. La microcapsule selon l'une quelconque des revendications 1 à 2, ou la microcapsule multicouche selon l'une quelconque des revendications 3 à 7, qui comprend en outre au moins un probiotique, et/ou au moins un postbiotique.

9. La microcapsule selon l'une quelconque des revendications 1 à 2, ou la microcapsule multicouche selon l'une quelconque des revendications 3 à 7, qui a une taille comprise de 50 à 500 µm.

10. Un procédé de préparation d'une microcapsule telle que définie dans l'une quelconque des revendications 1 à 2, ou telle que définie dans la revendication 8, comprenant :
i. préparer une solution comprenant de l'acide gamma-aminobutyrique (GABA), et facultativement au moins un probiotique, et/ou au moins un postbiotique, incorporé dans un polymère formant un hydrogel choisi parmi l'alginate et/ou la pectine, en dissolvant tout en agitant le GABA dans une solution comprenant le polymère formant un hydrogel dans un rapport pondéral de 1:1 à 1:3,0 de GABA:polymère formant un hydrogel, dans lequel, lorsque ledit polymère formant un hydrogel est l'alginate, il a un poids moléculaire tel qui donne une viscosité de 100 mPa.s à moins de 400 mPa.s, lorsqu'elle est mesurée à 25 °C dans un viscosimètre dynamique et dans une solution aqueuse à une concentration de 10 à 20 g/L dudit polymère d'alginate ;
ii. extruder la solution de l'étape (i) à travers une buse avec une section de 50 à 500 µm, tout en appliquant une fréquence de vibration aux buses de 100 Hz à 1000 Hz pour obtenir des gouttelettes ; et
iii. récolter les gouttelettes dans une solution d'ions divalents pour le durcissement ; et
iv. facultativement sécher les microcapsules obtenues à l'étape (iii).

11. Un procédé pour la préparation d'une microcapsule multicouche telle que définie dans l'une quelconque des revendications 3 à 8, comprenant :
(i) préparer une solution de noyau (a) comprenant de l'acide gamma-aminobutyrique (GABA), et facultativement au moins un probiotique, et/ou au moins un postbiotique, incorporé dans un polymère formant un hydrogel choisi parmi l'alginate et/ou la pectine, en dissolvant tout en agitant le GABA dans une solution comprenant le polymère formant un hydrogel dans un rapport pondéral de 1:1 à 1:3 de GABA:polymère formant un hydrogel, dans lequel, lorsque ledit polymère formant un hydrogel est l'alginate, il a un poids moléculaire tel qui donne une viscosité de 100 mPa.s à moins de 400 mPa.s, lorsqu'elle est mesuré à 25 °C dans un viscosimètre dynamique et dans une solution aqueuse à une concentration de 10 à 20 g/L dudit polymère d'alginate ;
(ii) préparer une solution de couche de revêtement (b) comprenant un polymère formant un hydrogel,
(iii) extruder la solution de noyau (a) et la solution de couche de revêtement (b) des étapes (i) et (ii) à travers de buses concentriques, chacune ayant une section de 50 à 500 µm et ayant une section ou des diamètres différents définissant une buse interne et une buse externe, tout en appliquant une vibration de fréquence de 100 Hz à 1000 Hz aux buses pour obtenir des gouttelettes multicouches, de telle sorte que la solution de noyau s'écoule à travers la buse interne et la solution de couche de revêtement s'écoule à travers la buse externe ; et
(iv) récolter les gouttelettes multicouches dans une solution d'ions divalents pour le durcissement ; et
(v) facultativement sécher les microcapsules obtenues à l'étape (iv).

12. Le procédé selon l'une quelconque des revendications 10 à 11, dans lequel la concentration en polymère dans les solutions (i) et (ii) est de 1 à 3 % (p/v) lorsque le polymère est l'alginate et de 3 à 7 % (p/v) lorsque le polymère est la pectine.

13. Le procédé selon l'une quelconque des revendications 10 à 12, dans lequel le probiotique, lorsqu'il est présent, est à une concentration comprise de 10⁷ à 10⁹ ufc/mL de solution et le postbiotique, lorsqu'il est présent, est à une concentration comprise de 0,01 à 0,05 g/mL de solution.

14. Un produit alimentaire ou d'alimentation animale, un ingrédient alimentaire ou d'alimentation animale, une composition nutraceutique, une composition pharmaceutique ou une composition cosmétique comprenant des microcapsules telles que définies dans l'une quelconque des revendications 1 à 9.
